# EUROPÄISCHE PATENTANMELDUNG

(11) **EP 0 721 767 A2**
(43) Veröffentlichungstag der Anmeldung: **17.07.1996**
(21) Anmeldenummer: 96250002.1
(22) Anmeldetag: 08.01.1996
(51) Int. Cl.: A61F 2/36

(54) **Endoprothese**

(30) Priorität: 11.01.1995 DE 19501995
(71) Anmelder: BIOMET Deutschland GmbH, D-12247 Berlin (DE)
(72) Erfinder: Goymann, Volkmar, Prof. Dr. med., D-45239 Essen (DE); Anapliotis, Emmanuel, D-14195 Berlin (DE); Darga, Jürgen, D-13407 Berlin (DE)
(74) Vertreter: Christiansen, Henning, Dipl.-Ing.

(57) **Zusammenfassung**

Die Erfindung betrifft eine Endoprothese für eine zementlose Verankerung, mit einem in Längsrichtung in einem Knochen zu verankernden Prothesenschaft, welcher am proximalen Ende einen Kragen mit einem Zapfen zur Aufnahme einer Gelenkkugel trägt, wobei der Prothesenschaft mehrere parallel zueinander angeordnete, miteinander verbundene, plattenförmig ausgebildete Lamellen aufweist und sein Querschnittsprofil eine offene Kontur bildet. Die Lamellen sind in Form eines Mittelsteges (4) und zwei den Mittelsteg (4) zumindest teilweise umgreifenden Seitenwandungen (2, 3) vorgesehen und am distalen Ende des Prothesenschaftes (20) schaufelförmig unter Bildung einer gemeinsamen Schneidkante (10) zusammengeführt.

## Beschreibung

Die Erfindung betrifft eine Endoprothese der im Oberbegriff des Anspruchs 1 angegebenen Art.

Es sind Hüftendoprothesen bekannt, die aus einem Prothesenkopf, Prothesenkragen und einem Prothesenschaft bestehen, der im Markraum des Femur eines Patienten zementlos verankert wird, nachdem der Markraum durch teilweises Entfernen der Spongosia entsprechend vorbereitet worden ist.

Bei zementloser Verankerung des Prothesenschaftes im Markraum des Femur wird eine Schaftstruktur mit vorzugsweise poröser Oberfläche verwendet, wodurch die möglichst feste Verankerung des Schafts im Markraum durch das Einwachsen von spongiösem Material unterstützt werden soll.

Die Entfernung der Spongiosa ist jedoch als unphysiologische lokale Störung im Sinne einer Defektbildung anzusehen. Derartige Defektbildungen führen zu einer Beeinträchtigung der Belastungsfähigkeit einer implantierten Endoprothese und vor allem der alterungsabhängigen Anpassungsmöglichkeiten.

Aus dem europäischen Patent EU-B1 0 295 200 ist eine Endoprothese bekannt, welche für eine Verankerung des Prothesenschaftes im Markraum des Femur ohne eine Entfernung der Spongiosa der regio intertrochanterica vorgesehen ist.

Die bekannte Endoprothese ist für eine zementlose Verankerung vorgesehen. Sie weist einen in Längsrichtung in einem Femurknochen zu verankernden Prothesenschaft auf, welcher am proximalen Ende einen Kragen mit einem Zapfen zur Aufnahme einer Gelenkkugel trägt. Der Prothesenschaft ist aus mehreren parallel zueinander angeordneten, miteinander verbundenen, plattenförmig ausgebildete Lamellen derart geformt, daß er eine im Querschnitt offene Kontur bildet. Die Lamellen sind mit entlang ihrer Peripherie verlaufenden Schneidkanten versehen.

Die vorstehend beschriebene Endoprothese weist jedoch den wesentlichen Nachteil auf, daß sich die distal vorgesehenen Schneidkanten im wesentlichen senkrecht zur Eintreibrichtung der Prothese erstrecken und dadurch einen erheblichen Kraftaufwand zum Eintreiben erfordern. Die relativ große Länge der distalen Schneidkanten bewirkt weiterhin in nachteiliger Weise, daß die Prothese im wesentlichen nur in dem intertrochantären Spongiosa-Bereich positioniert werden kann, ohne eine störende, medial oder lateral gerichtete Druckkraft im Knocheninneren hervorzurufen.

Daraus ergibt sich als weiterer Nachteil der bekannten Endoprothesen, daß eine ungleichmäßige Kraftverteilung durch eine im wesentlichen primär proximale Einleitung der Kräfte in den Oberschenkelteil eintritt, wodurch der distal vorgesehene Knochenanteil aus der funktionellen Belastung genommen wird. Dies führt zu einer Atrophie und nicht zu dem erwünschten funktionellen Reiz für den Knochen, fixierend an die Endoprothese heranzuwachsen.

Die Folge ist eine vorzeitige Lockerung der Endoprothese mit der Notwendigkeit einer Revisionsoperation. Eine solche Revisionsoperation ist jedoch für den Patienten in jedem Fall nachteilig, da ein Entfernen der Endoprothese nur mit einer erheblichen Zerstörung des Knochenmaterials möglich ist, was letztlich das Einwachsen einer neuen Endoprothese erschwert und zum Teil unmöglich macht.

Ein weiterer Nachteil bekannter Endoprothesen besteht darin, daß Standardmodelle für unterschiedliche Femurgrößen vorhanden sind, die aber nicht an die individuelle Form des proximalen Femur eines Patienten angepaßt sind, so daß die ungünstige Krafteinleitung vieler Endoprothesen noch verstärkt und die vorhandene Knochensubstanz nicht optimal genutzt wird.

Der Erfindung liegt die Aufgabe zugrunde, eine Endoprothese der eingangs genannten Art zu schaffen, die die Erhaltung der Physiologie des Knochenmarkraumes einschließlich der intertrochantären und der subtrochantären Spongiosa, eine vereinfachte Operationstechnik ohne die Notwendigkeit eines Vormeißelns oder Vorbohrens und eine optimale physiologische Krafteinleitung sicherstellt sowie gleichzeitig eine vergrößerte Anlagefläche für das Anwachsen von Knochenmaterial ermöglicht.

Diese Aufgabe wird mit dem kennzeichnenden Merkmal des Anspruchs 1 gelöst.

Der Erfindung schließt die Erkenntnis ein, daß das Anbringen einer Endoprothese unter Erhalt der spongiösen Strukturen weder die Stoffwechselbiologie wesentlich stört und noch die Durchblutungsbedingungen grundsätzlich beeinträchtigt. Durch den Erhalt der Physiologie des Knochenmarkraumes einschließlich der intertrochantären und subtronchantären Spongiosa werden günstige anatomische Bedingungen bei Revisionsoperationen und optimale Anpassungsmöglichkeiten an altersbedingte Knochenveränderungen geschaffen.

Die erfindungsgemäße Endoprothese ermöglicht eine sichere Verankerung der Lamellen, ohne daß die jeweils in die Spongiosa einzutreibenden Lamellen der Endoprothese eine zusätzliche Vorbereitung der Spongiosa-Struktur, beispielsweise ein Vorschneiden oder Raspeln, erforderlich machen. Die Formgebung der Lamellen führt in günstiger Weise zu einer Verringerung der zum Eintreiben der Prothese erforderlichen Kraft. Wesentliche Voraussetzung für das Eintreiben des Prothesenschaftes ist, daß alle einzutreibenden Teilflächen des Systems der Endoprothese parallel angeordnet sind und parallel zur Femurachse, d.h. senkrecht zum Querschnitt eingeschlagen werden.

Dieses Vorgehen bietet entscheidende operationstechnische Vorteile, da ein Raspeln oder Aufbohren nicht notwendig ist, was eine erhebliche Zeitersparnis und eine Minderung des Infektionsrisikos sowie die Vermeidung einer via falsa zur Folge hat.

Da sich die als plattenförmige Teilflächen ausgebildeten Lamellen bei eingeschlagenem Prothesenschaft bis in den subtrochantären Spongiosabereich erstrecken, wird zum einen eine vergrößerte Anlagefläche für das Anwachsen von Knochenmaterial geschaffen und zum anderen ist die Voraussetzung für eine günstige Krafteinleitung in den Femurknochen bei Belastung der implantierten Prothese gegeben.

Entsprechend der bevorzugten Ausführungsform der Erfindung weist der Prothesenschaft drei Lamellen auf, welche als Mittelsteg und zwei den Mittelsteg zumindest teilweise umgreifenden Seitenwandungen vorgesehen sind. Über den Mittelsteg erfolgt im wesentlichen die Krafteinleitung von dem die Gelenkkugel tragenden Zapfen in den Prothesenschaft. Um die Druckbelastung der Spongiosa bei Eintreiben des Prothesenschaftes in den Knochen weitestgehend zu minimieren, sind die Lamellen am distalen Ende des Prothesenschaftes schaufelförmig unter Bildung einer gemeinsamen Schneidkante zusammengefaßt, wobei sich alle Lamellen im wesentlichen gleichartig nach distal stromlinienförmig verjüngen.

Um für das Einbringen des Prothesenschaftes günstige Bedingungen zu schaffen, sind die medialen Begrenzungskanten der den Schaft bildenden Lamellen entsprechend einer Weiterbildung der Erfindung in wesentlichen S-förmig ausgebildet.

Der Prothesenschaft weist ein M-förmiges Querschnittprofil auf, wobei der Mittelschenkel des Profils die Profilaußenschenkel nach medial überragt. Durch die Positionierung der Schneidkante am distalen Ende des Schaftes und die Parallelführung der Lamellen können sich die Spongiosa-Abschnitte ohne wesentliche Zerstörung ihrer Struktur in dem von den Lamellen gegrenzten Freiraum bewegen, wobei die Sponiosa nur beim Eintreiben einmal auseinandergeschnitten wird, so daß sie sich danach mit dem größten Teil der Schnittfläche wieder exakt adaptieren kann.

Das durch die Form der erfindungsgemäßen Endoprothese mögliche Einsetzen der Prothese in den Femurknochen eines Patienten unter weitestgehender Schonung der Spongiosa schafft eine gesicherte physiologische, d.h. proximale Krafteinleitung, wobei die spongiösen Strukturen extrem festen Halt bieten und das Übertragen enormer Druckkräfte erlauben.

Um die Stabilität des Prothesenschaftes zu sichern sind die Schenkel des Schaftprofils durch schmale Stege miteinander verbunden. Dabei ist es besonders günstig, jeweils zwei der im wesentlichen mit gleichem Abstand über die gesamte Schaftlänge verteilt angeordneten und jeweils eine der Schaftseitenwandungen mit der mittleren Lamelle verbindenden Stege in gleicher Höhe zu positionieren und diese mit einer nach distal gerichteten Schneidkante zu versehen. Diese schmalen Verbindungsstege stabilisieren nicht nur den Prothesenschaft, sondern bieten gleichzeitig zusätzliche Verankerungsmöglichkeiten für die spongiösen Strukturen im inter- und subtrochantären Bereich des Knochens und erhöhen den Widerstand beim Eintreiben des Prothesenschaftes nur unwesentlich. Durch die schaufelförmige Ausgestaltung des beim Eintreiben vorangeführten Endes ist eine präzise Führung sichergestellt, welche dafür sorgt, daß eine Einbringen mit geringem Kraftaufwand erfolgen kann. Dies gilt insbesondere, da auch die lamellen der Struktur verbindene Querversteifungen mit Schneidkanten in Eintreibrichtung versehen sind.

Durch die mit quergerichteten Aussteifungen versehene lamellenartige Struktur wird dem Eintreiben lediglich der Materialquerschnitt der Wandungen als Widerstand entgegengestellt. Das sich beim Eintreiben zwischen den Lamellen einlagernde Körpergewebe bildet mit dem Prothesenmaterial eine Sandwichstruktur, welche eine hohe Steifigkeit gegenüber den auftretenden Belastungen aufweist. Durch die geschlossen verrundete Struktur der Rückseite bietet sich eine geschlossene Anlagefläche zur Krafteinleitung in die umgebende Knochenstruktur im Bereich der Hauptbelastung. Öffnung im Bereich der Seitenwandungen verringern die Masse und ermöglichen das Anwachsen bzw. das Anbringen von Zusatzelementen zur unsymmetrischen Anpassung zum Einsatz in der linken bzw. rechten Körperhälfte.

Entsprechend einer anderen vorteilhaften Ausführungsform der Erfindung sind am proximalen Ende des Prothesenschaftes Durchbrüche vorgesehen, welche sich parallel zueinander und zur Schaftachse nach distal erstrecken. Das distale Ende der Durchbrüche mündet in die zwischen den Lamellen des Schaftes vorhandenen, durch das M-förmige Querschnittprofil bestimmten Raumvolumina. Die Durchbrüche ermöglichen auf einfache Weise einen Druckausgleich während des Einbringens des Prothesenschafts in den Femurknochen und gestatten gleichzeitig den Durchtritt von gegebenenfalls vorhandener Körperflüssigkeit.

Eine weitere vorteilhafte Ausgestaltung der erfindungsgemäßen Lösung ist dadurch gekennzeichnet, daß die die Seitenwandungen des Schaftes bildenden Lamellen in ihrem proximalen Bereich Ausnehmungen und/oder Durchbrüche aufweisen. Dadurch werden zusätzliche Freiräume zum Einwachsen von spongiösem Material geschaffen, so daß zum einen eine komplex verzahnte Spongiosastruktur zur Weiterleitung der auftretenden Kräfte nutzbar ist und andererseits eine optimale Verbindung der Endoprothese mit dem Knochen sichergestellt wird, so daß Lockerungen nicht oder nur in geringem Maße zu befürchten sind. Für die Durchbrüche ist beispielsweise eine kreisförmige Ausbildung günstig, um zylindrische Stützelemente, vorzugsweise aus körpereignem, spongiösem Material oder eines bioporösen Fremdmaterial, einsetzen zu können, welche sich quer zur Achse des Prothesenschafts erstrecken und das Implantat durch Abstützen an der inneren Knochenwandung zusätzlich stabilisieren. Die Ausnehmungen in den Seitenwandungen sind vorzugsweise als Kreisabschnitt ausgebildet und erstrecken sich bis an die Lateralseite des Schaftes, so daß der Schaftquerschnitt von distal gegebenenfalls nur bis zur halben Schaftlänge M-förmig profiliert ist.

Eine zusätzliche Weiterbildung der Prothese weist in vorteilhafter Weise einen Schaft auf, dessen laterale Wandung mehrfach geschlitzt ausgebildet ist, wobei die einzelnen, sich parallel zur Schaftachse erstreckenden Schlitze durch eine proximale und eine distale Verbindung sowie eine Stegverbindung der Lamellen in der Schaftmitte entstehen. Eine derartige Schaftausbildung verbessert die Bedingungen für das Einwachsen des Implantats zusätzlich.

Die erfindungsgemäße Endoprothese besteht vorzugsweise aus Titan. Für ihre Herstellung ist die Anwendung eines Vakuum-Gußverfahrens besonders geeignet.

Für eine weitere Verbesserung des Einwachsens von spongiösem Material ist ein Beschichten der inneren Wandungsbereiche des in offener Kastenbauweise hergestellten Prothesenschaftes von Vorteil. Besonders günstig ist das Auftragen einer künstlichen Spongiosa in Form eines bioporösen Materials, beispielsweise Hydroxylapatit-Keramik, oder eines Plasmasprays mit einer Schichtdicke im Bereich von 50 bis 100 µm.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine Seitenansicht der bevorzugten Ausführungsform der Erfindung,
Figur 1a die Ansicht eines Schnittes längs der Linie A...A gemäß Figur 1,
Figur 2 die Darstellung der in Figur 1 gezeigten Prothese in Ansicht von links,
Figur 2a die Ansicht eines Schnittes längs der Linie B...B gemäß Figur 2,
Figur 3 die Darstellung der in Figur 1 gezeigten Prothese in Ansicht von oben,
Figur 4 eine günstige Weiterbildung der Erfindung in Ansicht von lateral
   sowie
Figur 4a eine Seitenansicht des proximalen Bereichs der in Figur 4 dargestellten Prothese.

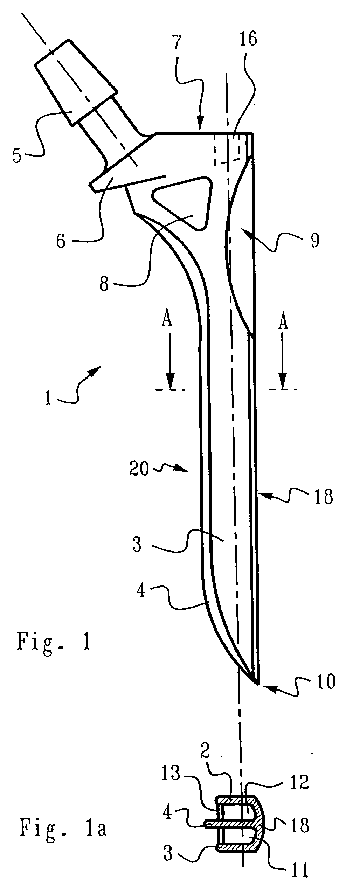

Die in den Figuren 1 und 1a dargestellte Endoprothese 1 weist einen aus drei parallel zueinander angeordneten und sich von proximal nach distal erstreckenden Lamellen 2, 3, 4 bestehenden Schaft 20. Die Lamellen 2, 3, 4 gehen proximal in einen Prothesenkragen 6 über, welcher einen Zapfen 5 zur Aufnahme einer (nicht dargestellten) Gelenkkugel trägt.

Der Prothesenschaft 20 weist als Querschnittsprofil eine offenen Kontur in Form eines M auf, wobei die Lamellen in Form eines Mittelsteges 4 und zwei den Mittelsteg 4 zumindest teilweise umgreifenden Seitenwandungen 2, 3 vorgesehen sind. Die lateralen Begrenzungskanten der Lamellen 2, 3, 4 gehen in die sich im wesentlichen geradlinig von proximal nach distal erstreckende laterale Schaftwandung 18 über, wogegen sich der Schaft 20 nach proximal stromlinienförmig erweitert und sich nach distal stromlinienartig verjüngt, wobei die medialen Begrenzungskanten der Lamellen im wesentlichen S-förmig ausgebildet sind. Um die Schaftprothese 1 ohne Vorbereiten des Markraumes durch Bohren oder Ausraspeln ohne besonderen Kraftaufwand in den Knochen einbringen zu können, sind die medialen Begrenzungskanten der Lamellen 2, 3, 4 am distalen Ende des Prothesenschaftes 20 schaufelförmig unter Bildung einer gemeinsamen Schneidkante 10 zusammengeführt. Die Spongiosa wird dabei in Längsrichtung schneidend geteilt und füllt die Freiräume 12, 13 innerhalb des Schaftquerschnitts.

Durch die relativ geringe Wandstärke der Lamellen und die Elastizität der Spongiosa bedingt, ergibt sich ein guter Kontakt zwischen Prothesenschaft 2 und Knochenmaterial, wodurch ein für die Belastbarkeit der Prothese erforderliches sicheres Einwachsen ermöglicht wird.

Die im proximalen Schaftbereich der Prothese 1 vorhandenen Durchbrüche bzw. Ausnehmungen 8, 9 in den Seitenwandungen 2, 3 sowie die über die Schaftlänge verteilt vorgesehenen Stege 13 schaffen zusätzliche Verankerungsmöglichkeiten für die Spongiosa. Die Durchbrüche 8 sind als Dreieicke mit verrundeten Kanten ausgebildet. Die kreisabschnittförmigen Ausnehmungen 9 erstrecken sich von der lateralen Schaftseite nach medial und schaffen ein beidseitig offenes Querschnittsprofil im proximalen Abschnitt des Schaftes 20.

Der Prothesenkragen 6 weist am proximalen Ende 7 des Schaftes 20 zwei parallel zueinander angeordnete Durchbrüche 16 auf, welche sich von proximal nach distal in Richtung der Schaftachse erstrecken. Das distale Ende der Durchbrüche 16 mündet in die zwischen den Lamellen 2, 3, 4 des Schaftes 20 vorhandenen, durch das M-förmige Querschnittprofil bestimmten Raumvolumina 11 und 12. Die Durchbrüche 16 ermöglichen auf einfache Weise einen Druckausgleich während des Einbringens des Prothesenschafts 20 in den Femurknochen und gestatten gleichzeitig den Durchtritt von gegebenenfalls vorhandener Körperflüssigkeit.

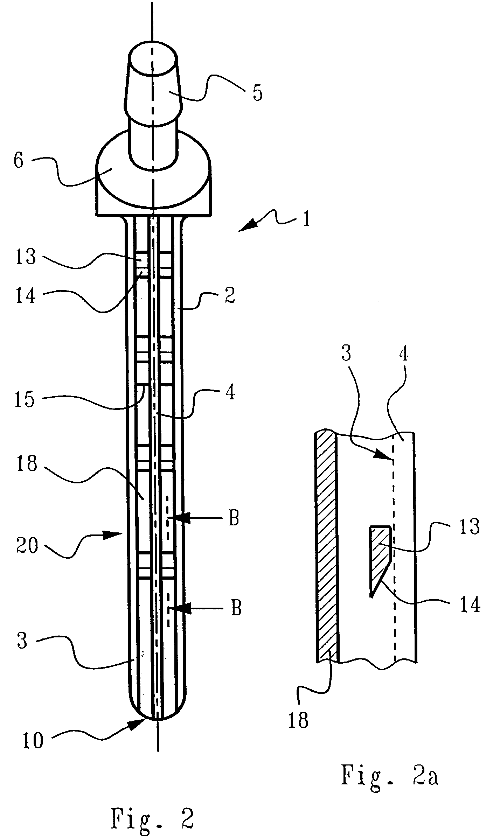

In Figur 2 ist die erfindungsgemäße Endoprothese 1 in Ansicht von medial dargestellt. Die die Lamellen 2, 3, 4 verbindenden Stege 13 sind gleichmäßig über die Länge des Schaftes 20 verteilt. Sie erstrecken sich quer zur Schaftachse und verbinden - wie in Figur 1a ersichtlich - die freien Enden der das M-Profil bildenden Seitenwände 2, 3 des Schaftes 20 mit dem Mittelsteg 4, wodurch die Stabilität des Schaftes erhöht wird und zusätzliche Verankerungsmöglichkeiten für die spongiösen Strukturen im inter- und subtrochantären Bereich des Knochens entstehen. Dabei ist es besonders günstig, jeweils zwei der im wesentlichen mit gleichem Abstand über die gesamte Schaftlänge verteilt angeordneten und jeweils eine der Schaftseitenwandungen 2, 3 mit der mittleren Lamelle 4 verbindenden Stege 13 in gleicher Höhe zu positionieren. Die Stege 13 sind mit einer nach distal gerichteten Schneidkante 14 versehen, so daß sie den Widerstand beim Eintreiben des Prothesenschaftes 20 in den Knochen nur unwesentlich erhöhen. Eine derartige Ausbildung der Stege und die schaufelförmige Schneidkante 10 am distalen Ende des Schaftes ermöglichen in günstiger Weise eine Minimierung der Belastung des Femur beim Eintreiben des Prothesenschaftes in die Spongiosa-Struktur des Knochens. Die Form der Schneidkante 14 ist gemäß Figur 2a in der Ansicht eines Schnittes längs der Linie B...B gemäß Figur 2 dargestellt.

Der Prothesenschaft 20 weist - bedingt durch die Ausnehmungen im proximalen Bereich (vergleiche Position 9 gemäß Figur 1) - keine geschlossene Lateralwandung 18 auf. Die entsprechende Wandungskante 15 befindet sich oberhalb der Schaftmitte. Dadurch können die spongiösen Strukturen des Knochens sowohl die durch die Lamellen 2, 3 gebildeten Seitenwandungen als auch den Mittelsteg 4 des Schaftes 20 in deren proximalen Abschnitten vollständig umgreifen, wodurch sich die Festigkeit des Implantats im Knochen weiter erhöht.

Der Mittelsteg 4 des Schaftes 20 ist über den Prothesenkragen 6 mit dem zur Aufnahme einer Gelenkkugel vorgesehenen Zapfen 5 verbunden und bietet damit günstige Bedingungen für eine zentrale Krafteinleitung bei belasteter Prothese.

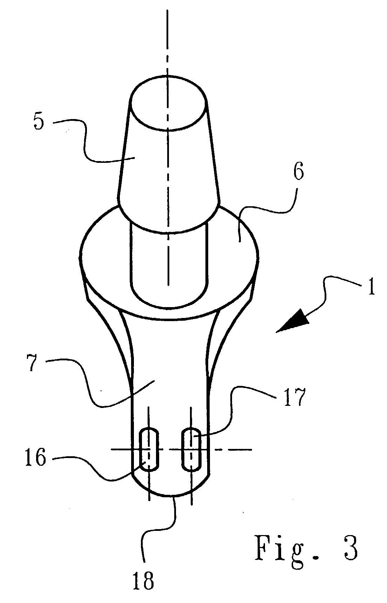

Die in den Figuren 1 und 2 dargestellte Endoprothese 1 ist in Figur 3 in Ansicht von oben gezeigt. Am proximalen Ende 7 des Prothesenschafts sind an der den Zapfen 5 tragenden Prothesenkragen 6 abgewandten Seite zwei Durchbrüche 16, 17 vorgesehen, welche mit den Raumvolumina zwischen den Schaftlamellen (vergleiche die Positionen 11, 12 sowie 2, 3, 4 gemäß Figur 1 und Figur 1a) verbunden sind. Die Lateralwandung des Prothesenschaftes ist mit 18 bezeichnet. Die Durchbrüche 16, 17 erstrecken sich parallel zueinander und zur Schaftachse nach distal. Die Durchbrüche 16, 17 ermöglichen in günstiger Weise einen Druckausgleich während des Einbringens des Prothesenschafts in den Femurknochen und gestatten gleichzeitig den Durchtritt von gegebenenfalls vorhandener Körperflüssigkeit.

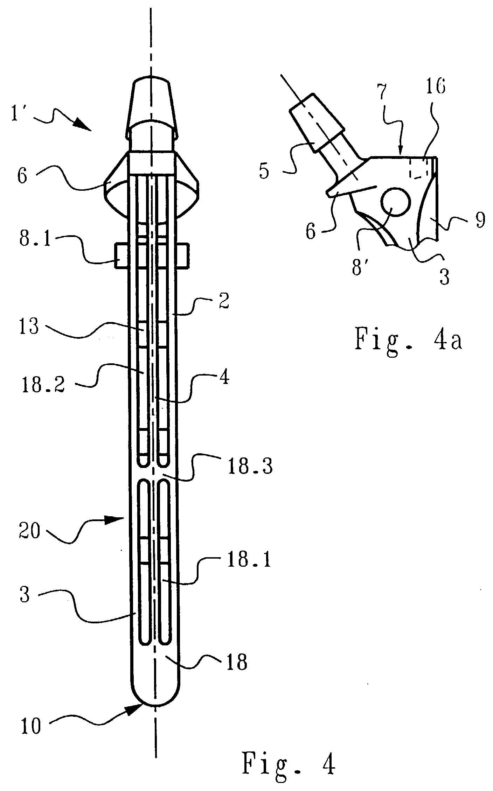

Der Schaft 20 der in Figur 4 in Ansicht von lateral dargestellten Prothese 1' weist in seiner Lateralwandung 18 sich von proximal nach distal erstreckende Schlitze 18.1 und 18.2 auf. Diese Schlitze entstehen dadurch, daß die Schaftlamellen 2, 3 und 4 nur an ihrem distalen Ende in Form einer schaufelförmigen Schneide 10, in der Schaftmitte durch einen schmalen Steg 18.3 und proximal durch den Prothesenkragen miteinander verbunden sind. In Verbindung mit den Schneidkanten (vergleiche die Position 14 gemäß Figur 2a) aufweisen stegförmigen Brücken 13 an der medialen Sete des Schaftes 20 ist durch den Steg 18.3 eine ausreichende Steifigkeit des Prothesenschaftes gesichert. Die schlitzförmige Gestaltung der Schaftwandung 18 führt zu einer weiteren Verbesserung der Bedingungen für das Einwachsen der Prothese und damit zu höherer Belastbarkeit des Implantats.

Der in Figur 4a dargestellte proximale Abschnitt der Prothese gemäß Figur 4 zeigt in der Seitenansicht einen kreisförmig ausgebildeten Durchbruch 8' in der Schaftseitenwand 3. Die Durchbrüche in den Seitenwänden des Schaftes sind in günstiger Weise für das Einsetzen zylindrischer Stützelemente (vergleiche die Positionen 2, 3 und 8.1 gemäß Figur 4) nutzbar, mit welchen sich die implantierte Prothese beidseitig an der inneren Knochenwandung abstützen kann. Stützelemente aus körpereigener Spongiosa oder bioporösem Fremdmaterial, beispielsweise Hydroxylapatit-Keramik, fördern sowohl ein sicheres Einwachsen der Endoprothese und ermöglichen eine besonders hohe mechanische Belastbarkeit des Implantats.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Endoprothese für eine zementlose Verankerung, mit einem in Längsrichtung in einem Knochen zu verankernden Prothesenschaft, welcher am proximalen Ende einen Kragen mit einem Zapfen zur Aufnahme einer Gelenkkugel trägt, wobei der Prothesenschaft mehrere parallel zueinander angeordnete, miteinander verbundene, plattenförmig ausgebildete Lamellen aufweist und sein Querschnittsprofil eine offene Kontur bildet,
**dadurch gekennzeichnet,**
daß die Lamellen in Form eines Mittelsteges (4) und zwei zum Mittelsteg (4) sich zumindest teilweise parallel erstreckenden Seitenwandungen (2, 3) vorgesehen und mindestens am distalen Ende des Prothesenschaftes (20) schaufelförmig unter Bildung einer gemeinsamen Schneidkante (10) zusammengeführt sind.

2. Endoprothese nach Anspruch 1, **dadurch gekennzeichnet**, daß der Mittelsteg (4) durch den Prothesenkragen (6) mit dem Zapfen (5) verbunden ist.

3. Endoprothese nach Anspruch 1, **dadurch gekennzeichnet**, daß sich die Lamellen (2, 3, 4) nach distal unter Verrundung stromlinienförmig verjüngen.

4. Endoprothese nach Anspruch 3, **dadurch gekennzeichnet**, daß die mediale Begrenzungskante der Lamellen (2, 3, 4) im wesentlichen S-förmig ausgebildet ist.

5. Endoprothese nach Anspruch 1, **dadurch gekennzeichnet**, daß das Querschnittprofil des Schaftes (20) im wesentlichen M-förmig ausgebildet ist.

6. Endoprothese nach Anspruch 5, **dadurch gekennzeichnet**, daß der Mittelsteg (4) des M-förmigen Profils die Profilseitenstege (2, 3) nach medial überragend ausgebildet ist.

7. Schaftprothese nach Anspruch 1, **dadurch gekennzeichnet**, daß der Mittelsteg (4) mit den Seitenstegen (2, 3) durch sich quer zur Längsausdehnung des Schaftes (20) erstreckende Brücken (13) verbunden ist.

8. Endoprothese nach Anspruch 7, **dadurch gekennzeichnet**, daß die Brücken (13) stegartig ausgebildet sind und jeweils eine nach distal gerichtete Schneidkante (14) aufweisen.

9. Endoprothese nach einem der vorangehenden Ansprüche, **dadurch gekennzeichnet**, daß der Prothesenschaft (20) an seinem proximalen Ende (7) Durchbrüche (16, 17) aufweist, welche mit den durch die Lamellen (2, 3, 4) begrenzten Raumvolumina (11, 12) verbunden sind.

10. Endoprothese nach Anspruch 9, **dadurch gekennzeichnet**, daß jedem Raumvolumen (11, 12) ein Durchbruch (16, 17) zugeordnet ist.

11. Endoprothese nach Anspruch 10, **dadurch gekennzeichnet**, daß die Durchbrüche (16, 17) als sich von medial nach lateral erstreckende Langlöcher ausgebildet sind.

12. Endoprothese nach Anspruch 11, **dadurch gekennzeichnet**, daß die Langlöcher (16, 17) zueinander parallelliegend angeordnet sind und verrundete Kanten aufweisen.

13. Endoprothese nach einem der vorangehenden Ansprüche, **dadurch gekennzeichnet**, daß die den Mittelsteg (4) umgebenden Seitenwandungen (2, 3) im proximalen Bereich jeweils mindestens einen Durchbruch (8) und eine Ausnehmung (9) aufweisen.

14. Endoprothese nach Anspruch 13, **dadurch gekennzeichnet**, daß die Ausnehmung (9) als sich nach lateral öffnender Kreisabschnitt ausgebildet ist, wogegen die Durchbrüche (8, 8') eine Dreieck- oder Kreisform aufweisen.

15. Endoprothese nach Anspruch 14, **dadurch gekennzeichnet**, daß in die Durchbrüche (8, 8') sich quer zur Schaftachse erstreckende Stützelemente (8.1) eingesetzt sind.

16. Endoprothese nach Anspruch 15, **dadurch gekennzeichnet**, daß die Stützelemente (8.1) aus spongiösem oder bioporösem Material bestehen.

17. Endoprothese nach einem der vorangehenden Ansprüche, **dadurch gekennzeichnet**, daß der Prothesenschaft (20) in seinem proximalen Bereich eine nur teilweise geschlossene Lateralwandung (18) aufweist.

18. Endoprothese nach Anspruch 17, **dadurch gekennzeichnet**, daß in der Lateralwandung (18) des Schaftes (20) sich parallel zur Schaftachse erstreckende Längsschlitze (18.1, 18.2) vorgesehen sind.

19. Endoprothese nach einem der vorangehenden Ansprüche, **gekennzeichnet durch** eine Herstellung im Vakuumguß-Verfahren, wobei als Werkstoff vorzugsweise Titan vorgesehen ist.

20. Endoprothese nach einem der vorangehenden Ansprüche, **dadurch gekennzeichnet**, daß die durch die Lamellen (2, 3, 4) gebildeten Wandungsbereiche des Prothesenschaftes (20) eine durch Sandstrahlen erzeugte Rauhigkeit aufweisen oder eine Beschichtung aus bioporösem Material tragen.

21. Endoprothese nach Anspruch 20, **dadurch gekennzeichnet**, daß die Beschichtung aus einer Hydroxylapatit-Keramik oder einem Plasma-Spray mit einer Schichtdichte im Bereich von 50 bis 100 µm besteht.
